# EUROPÄISCHE PATENTSCHRIFT

(11) **EP 0 778 261 B1**
(45) Veröffentlichungstag und Bekanntmachung des Hinweises auf die Patenterteilung: **30.09.1998**
(21) Anmeldenummer: 96118858.8
(22) Anmeldetag: 26.11.1996
(51) Int. Cl.: C07C 209/72

(54) **Verfahren zur Herstellung eines Gemisches aus Cyclohexylamin und Dicyclohexylamin**
Process for the preparation of a cyclohexylamine and dicyclohexylamine mixture
Procédé pour la préparation d'un mélange de cyclohexylamine et de dicyclohexylamine

(30) Priorität: 08.12.1995 DE 19545884
(43) Veröffentlichungstag der Anmeldung: 11.06.1997
(73) Patentinhaber: BAYER AG, 51368 Leverkusen (DE)
(72) Erfinder: Darsow, Gerhard, Dr., 47804 Krefeld (DE); Petruck, Gerd-Michael, Dr., 40699 Erkrath (DE); Niemeier, Wilfried, Dr., 47803 Krefeld (DE)

(56) Entgegenhaltungen:
- EP-A- 0 053 818
- EP-A- 0 503 347
- GB-A- 969 542
- US-A- 4 057 513
- US-A- 4 914 239
- PATENT ABSTRACTS OF JAPAN vol. 016, no. 176 (C-0934), 27.April 1992 & JP 04 018935 A (KAWAKEN FINE CHEM CO LTD), 23.Januar 1992,

## Beschreibung

Die Erfindung betrifft ein Verfahren zur Herstellung eines Gemisches von gegebenenfalls substituiertem Cyclohexylamin und gegebenenfalls substituiertem Dicyclohexylamin in variablen Mengen durch katalytische Flüssigphasenhydrierung von gegebenenfalls substituiertem Anilin mit Wasserstoff bei erhöhter Temperatur unter Einsatz eines Systems von zwei reduzierten, trägerlosen verpreßten oxidischen Festbettkatalysatoren. Gegebenenfalls substituierte Cyclohexylamine und Dicyclohexylamine finden Verwendung zur Herstellung von Alterungsschutzmitteln für Kautschuke und Kunststoffe, als Korrosionsschutzmittel in wäßriger Lösung, sowie als Vorprodukte für Textilhilfsmittel und Pflanzenschutzmittel.

Es ist bekannt, Cyclohexylamin durch Druckhydrierung von Anilin herzustellen (US-A-4 914 239). Für diese Hydrierung werden hauptsächlich kostspielige Edelmetall-Katalysatoren eingesetzt, beispielsweise gemäß US-A-3 636 108 ein mit Alkali moderierter Ru-Katalysator, wobei noch zusätzlich NH₃ und gegebenenfalls ein Lösungsmittel eingesetzt werden. Ein weiteres Verfahren zur Druckhydrierung von Anilin zu Cyclohexylamin ist in DE-B-1 106 319 beschrieben, wobei ebenfalls ein Ru-Katalysator verwendet wird. In diesem Verfahren wird mitentstehendes Dicyclohexylamin dem Einsatzstoff wieder zugesetzt. Das Verfahren erreicht jedoch wegen der gleichzeitigen Entstehung von Cyclohexan nur eine mäßige Ausbeute. Nach EP-B-53 818 sind Pd-Trägerkatalysatoren günstiger als Ru-Katalysatoren; die dort beschriebenen Katalysatoren enthalten Zusätze, die entweder einer Gruppe von basischen Verbindungen der Alkalimetalle, Erdalkalimetalle und Seltenerdmetalle entstammen oder einer Gruppe, welche die Metalle Fe, Ni, Co, Mn, Zn, Cd und Ag umfaßt. Diese Katalysatoren erlauben die Reduktion von substituierten Anilinen zu den zugehörigen Cyclohexylaminen; die zugehörigen Dicyclohexylamine fehlen jedoch völlig. Dies gilt gleichermaßen für Co-Katalysatoren, die einen basischen Zusatz enthalten (GB-A-969 542) sowie für Raney-Co (JP 68/03 180).

Bei den beschriebenen Druckhydrierungsverfahren von Anilin entsteht das Dicyclohexylamin neben dem Cyclohexylamin lediglich als Nebenprodukt oder überhaupt nicht. Um Dicyclohexylamin in größeren Mengen zu erhalten, wird es nach separaten Verfahren hergestellt. So kann es beispielsweise durch Druckhydrierung von Diphenylamin unter Verwendung eines Ru/Al₂O₃-Katalysators gewonnen werden (obige DE-B 1 106 319). Weiterhin entsteht Dicyclohexylamin bei der Umsetzung von Cyclohexanon mit Cyclohexylamin in Gegenwart von Pd auf Kohle unter einem Wasserstoffdruck von 4 bar (FR 1 530 477).

In einem umständlichen Verfahren kann Dicyclohexylamin aus dem Hydrierprodukt des Anilins an einem Ni-Katalysator durch fraktioniertes Auskondensieren gewonnen werden. Aus dem zurückbleibenden Gemisch wird ein Teil des mitentstandenen Ammoniaks entfernt und der Rest wieder in die Reaktion zurückgeführt (DE-C 805 518).

Ein gemeinsames Problem aller dieser Verfahren zur Kernhydrierung aromatischer Amine besteht in der z.T. beträchtlichen Bildung von Cyclohexan als nicht weiter zu verwendendem Nebenprodukt. Es bestand daher nach wie vor der Wunsch, ein neues, auch im technischen Maßstab brauchbares Verfahren zu entwickeln, nach welchem sowohl Cyclohexylamin als auch Dicyclohexylamin in einem gewünschten Mengenverhältnis hergestellt werden können, bei welchem der Verlust durch die unerwünschte Bildung von Cyclohexan zurückgedrängt wird und bei welchem weiterhin die Lebensdauer des verwendeten Katalysators verbessert ist.

Aus EP-A 501 265 ist ein Verfahren bekannt, gegebenenfalls substituiertes Cyclohexylamin und gegebenenfalls substituiertes Dicyclohexylamin durch katalytische Hydrierung von gegebenenfalls substituiertem Anilin herzustellen, wobei ein Katalysator eingesetzt wird, der Ru, Pd oder ein Gemisch beider Metalle enthält, die auf einem Träger aus Niobsäure oder Tantalsäure oder auf einem Gemisch beider aufgebracht sind. Aus EP-A 503 347 ist ein weiteres Verfahren bekannt, gegebenenfalls substituiertes Cyclohexylamin und gegebenenfalls substituiertes Dicyclohexylamin durch Hydrierung eines entsprechend substituierten Anilins herzustellen, wobei ein Katalysator eingesetzt wird, bei dem ein α- oder γ-Al₂O₃ als Träger zunächst mit mindestens einer Verbindung von Seltenen Erdmetallen und mit mindestens einer Verbindung des Mangans behandelt wird und danach mit mindestens einer Pd-Verbindung behandelt wird. Die Herstellung der Katalysatoren in diesen Verfahren von EP-A 501 265 und EP-A 503 347 ist technisch aufwendig und ihr Einsatz teuer, da die Wiedergewinnung der Edelmetallanteile aus den komplexen Substanzgemischen nach Erschöpfung des Katalysators erhebliche Probleme schafft, die anfangs nicht erkannt worden waren. Außerdem ist in diesem Verfahren das Mengenverhältnis der herstellbaren cyclischen Amine zu sehr in die Richtung höherer Anteile an Dicyclohexylamin verschoben. Schließlich ist die Lebensdauer der in den zuletzt genannten Verfahren eingesetzten Katalysatoren mit 3 000 bis 4 000 h zu gering, so daß sie die in sie gesetzten Erwartungen nicht erfüllen konnten.

Überraschenderweise wurde jetzt gefunden, daß die obengenannten Anforderungen durch den Einsatz eines Systems aus zwei preiswerten oxidischen Festbettkatalysatoren, die frei sind von inaktivem Trägermaterial und daher einfach aufgearbeitet und entsorgt werden können, erfüllt werden.

Die Erfindung betrifft somit ein Verfahren zur Herstellung eines Gemisches aus Cyclohexylamin und Dicyclohexylamin der Formeln 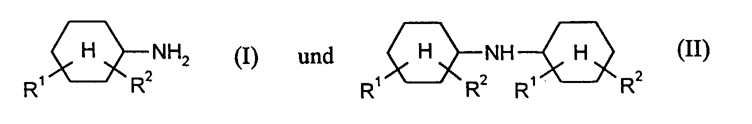 durch katalytische Hydrierung von Anilin der Formel 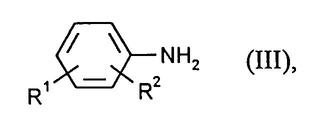 wobei in den Formeln
- R¹ und R²: unabhängig voneinander Wasserstoff, C₁-C₄-Alkyl oder C₁-C₄-Alkoxy bedeuten,
bei einer Reaktionstemperatur von 140 bis 260°C und einem H₂-Druck von 10 bis 400 bar, das dadurch gekennzeichnet ist, daß ein System aus zwei reduzierten, trägerlosen Katalysatoren A + B aus verpreßten Element(hydr)oxid-Pulvern eingesetzt wird, wobei folgende Elementanteile enthalten sind:
- für Katalysator A:: 40-60 Gew.-% eines oder mehrere von Fe, Co, Ni;
10-20 Gew.-% Mn; 0,05-1,5 Gew.-% Cu; und
0,2-5 Gew.-% eines oder mehrere von Ca, Sr, Ba; und
- für Katalysator B:: 30-50 Gew.-% eines oder mehrere von Fe, Co, Ni; 3-10 Gew.-% Mn; 5-15 Gew.-% Si; und 2-8 Gew.-% Mg;
und wobei in A und in B der Rest zu 100 Gew.-% Sauerstoff darstellt und die Prozentangaben auf das Gesamtgewicht des jeweiligen Katalysators A oder B bezogen sind.

Aus der Elementgruppe Fe, Co, Ni wird das Co oder das Ni oder ein Gemisch Co/Ni bevorzugt eingesetzt und das Co besonders bevorzugt eingesetzt.

Aus der Gruppe Ca, Sr, Ba wird das Sr oder das Ba oder ein Gemisch Sr/Ba bevorzugt eingesetzt und das Ba besonders bevorzugt eingesetzt.

Zur Herstellung der Katalysatoren A und B werden Pulver von Oxiden der genannten Elemente eingesetzt. Die aufgeführten Erdalkalimetalle können auch in Form ihrer Hydroxide eingesetzt werden. Das Si kann auch als Kieselgel eingesetzt werden. Bei den Schwermetallen ist es ebenfalls möglich, anstelle der Oxidpulver Hydroxidpulver einzusetzen, die entweder als solche zum Einsatz kommen oder nach Fällung aus wäßrigen Lösungen von Metallsalzen in bekannter Weise einzeln oder gemeinsam als Hydroxide gewonnen werden. In bevorzugter Weise werden Oxidpulver der genannten Elemente eingesetzt. Solche Pulver werden in den Mengen mechanisch miteinander vermischt, daß sie die oben angegebenen Gewichtsverhältnisse erfüllen. Der Rest zu 100 Gew.-% ist stets der Anteil des Sauerstoffs, und alle Gewichtsprozente sind auf das Gesamtgewicht des oxidischen, trägerfreien Preßlings bezogen. Das Gemisch der Pulver wird sodann auf Tablettier- oder Pelletier-Maschinen unter hohem Druck verpreßt, wobei zur Verbesserung des Haftvermögens der Pulver auch Graphit, Klebstoffe oder beide in Mengen von 0,5-1 Gew.-%, bezogen auf das Gesamtgewicht der zu verpressenden Pulver, zum Einsatz kommen können. Beispiele für die Form solcher Preßlinge sind Tabletten, Kugeln oder zylindrische Granulate mit Abmessungen von 1-10 mm, bevorzugt 3-7 mm. Tablettierte Formkörper können weiterhin zur Vergrößerung der äußeren Oberfläche mit einer axialen Durchbohrung versehen sein. Solche verpreßten Formkörper haben makroskopisch betrachtet eine glatte Oberfläche. Die verpreßten Formkörper haben eine hohe Druckfestigkeit auf die Formkörperoberfläche. So haben Tabletten oder zylindrische Granulate auf die ebenen Preßoberflächen eine Druckfestigkeit von 200-800 N/cm², bevorzugt 250-600 N/cm², und Tabletten, Kugeln oder zylindrische Granulate haben eine Druckfestigkeit auf die gewölbten Preßoberflächen von 50-200 N (gemessen als Kraft), bevorzugt 80-140 N. Die innere Oberfläche der eingesetzten verpreßten Formkörper beträgt 30-200 m²/g, bevorzugt 80-160 m²/g. Die Druckfestigkeit der trägerfreien Formkörper kann nach DIN 50 106 bestimmt werden. Die Bestimmung der inneren Oberfläche erfolgt nach F.M. Nelsen und F.T. Eggertsen, Analyt. Chem. 30 (1958), S. 1387-1390 bzw. nach S. J. Gregg und K.S.W. Sing, Adsorption Surface Area and Porosity, Academic Press, London 1982, Kap. 2 + 6.

Mit Hilfe des Einsatzes der beschriebenen Katalysatoren entsteht im erfindungsgemäßen Verfahren ein Gemisch von gegebenenfalls substituiertem Cyclohexylamin und gegebenenfalls substituiertem Dicyclohexylamin, wobei überraschend in Abhängigkeit von der Hydriertemperatur das Mengenverhältnis beider Amine so verändert werden kann, daß mit steigender Temperatur mehr gegebenenfalls substituiertes Dicyclohexylamin und weniger gegebenenfalls substituiertes Cyclohexylamin gebildet wird und mit sinkender Temperatur der umgekehrte Effekt erreicht wird.

Der Temperaturbereich für das erfindungsgemäße Verfahren beträgt 140-260°C, bevorzugt 160-230°C. Es wird bei einem H₂-Druck von 10-400 bar, bevorzugt 20-350 bar, besonders bevorzugt 100-300 bar, gearbeitet.

Das erfindungsgemäße Verfahren kann beispielsweise diskontinuierlich in einem Autoklaven oder kontinuierlich in der Rieselphase, jedenfalls aber in der Flüssigphase gearbeitet werden. Für technische Zwecke wird in bevorzugter Weise kontinuierlich gearbeitet, wobei die Katalysatorschüttungen fest angeordnet sind. Die Hydrierreaktoren für eine kontinuierliche Rieselphasenreaktion können einzelne Hochdruckrohre aus Stahl oder einer Stahllegierung sein, die mit den Formkörpern ganz oder teilweise gefüllt werden, wobei bei größeren Rohrquerschnitten auch die Anwendung der trägerfreien Formkörper auf Horden, wie Drahtkörben oder ähnlichen Einbauten, nützlich sein kann. Weiterhin kann man auch Hochdruck-Rohrbündel innerhalb eines gemeinsamen Mantels anwenden, wobei die Einzelrohre wiederum ganz oder teilweise mit dem Katalysatorformkörpern gefüllt werden.

Die genannten Katalysatoren A und B können grundsätzlich in Form einer Mischung eingesetzt werden; dies ist bei diskontinuierlicher Autoklavenfahrweise auch die einzig sinnvolle Form. Das Gewichtsverhältnis kann von 0,5 A:9,5 B bis 9,5 A:0,5 B eingestellt werden. Bei der Durchführung einer Rieselphasenhydrierung hat es sich als vorteilhaft herausgestellt, daß der zu hydrierende Ausgangsstoff zunächst eine Aufschüttung des Katalysators A passiert und danach eine Aufschüttung des Katalysators B. Hierbei können A und B in separat eingefüllten Schichten im gleichen Hochdruckrohr oder in zwei hintereinander geschalteten unterschiedlich befüllten Hochdruckrohren angeordnet seien. Auch bei einer solchen Anordnung gilt das genannte Gewichtsverhältnis von A:B. Grundsätzlich ist es jedoch auch möglich, beim Rieselphasenverfahren zunächst den Katalysator B und erst danach den Katalysator A zu passieren. Ebenso ist es grundsätzlich auch im Rieselphasenverfahren möglich, ein Gemisch von A und B einzusetzen.

Die verpreßten, trägerlosen Katalysatoren A und B werden durch Wasserstoff reduziert und damit aktiviert. Dies ist grundsätzlich simultan zur Hydrierung des eingesetzten Ausgangsmaterials möglich, wobei jedoch eine längere Einlaufphase erforderlich ist, bevor die Katalysatoren ihre volle Aktivität erreichen und damit die höchstmögliche Raum-Zeit-Ausbeute eintritt. Es ist daher vorteilhaft, beide Katalysatoren A und B vor der Beschickung mit Ausgangsmaterial getrennt oder gemeinsam zu reduzieren. Diese aktivierende Reduktion mit Wasserstoff wird im Temperaturbereich von 160-240°C und im Druckbereich von 10-400 bar durchgeführt. Hierbei wird zunächst durch ein Inertgas, wie Stickstoff, Argon, Methan oder Ethan der zunächst vorhandene Luftsauerstoff vollständig entfernt, bevor dem Inertgas ein Anteil von 10-15 Vol.-% Wasserstoff zugesetzt wird. Das Inertgas ist aus Gründen der guten Verfügbarkeit bevorzugt Stickstoff. Innerhalb eines festgesetzten Zeitraums, beispielsweise von 24 h wird sodann der Anteil an Inertgas ständig vermindert und schließlich das Inertgas ganz entfernt, so daß mit reinem Wasserstoff aktiviert und reduziert wird. Die Reduktion ist beendet, wenn der Katalysator keinen Wasserstoff mehr verbraucht und infolge dessen kein Reaktionswasser mehr bildet.

Bei der Verfahrensweise in Rieselphase beträgt die Katalysatorbelastung 0,1-3 kg, bevorzugt 0,15-1,5 kg, gegebenenfalls substituiertes Anilin pro Liter Katalysator und Stunde. Das eingesetzte, gegebenenfalls substituierte Anilin kann mit einem geeigneten reaktionsinerten Lösungsmittel, beispielsweise mit Cyclohexan oder Cyclohexanol in einer Menge von 10-100, bevorzugt 10-40 Gew.-%, bezogen auf das Gewicht des gegebenenfalls substituierten Anilins, verdünnt werden. Bei der kontinuierlichen Fahrweise in der Rieselphase kann es günstig sein, das eingesetzte, gegebenenfalls substituierte Anilin, nicht vollständig zu hydrieren, sondern einen Umsetzungsgrad von 80-97 % anzusteuern.

Das erfindungsgemäß eingesetzte System aus den reduzierten Katalysatoren A und B zeigt sehr hohe Standzeiten; bisher sind 25 000 bis 30 000 h beobachtet worden, bei denen Versuche ohne erkennbares Nachlassen der Aktivität abgebrochen wurden. Diese Standzeiten sind um ein Vielfaches höher, als sie in den obengenannten EP-A 501 265 und EP-A 503 347 beschrieben sind.

Als Einsatzmaterial für das erfindungsgemäße Verfahren kommen gegebenenfalls substituierte Aniline der obigen Formel (III) in Frage. C₁-C₄-Alkyl und C₁-C₄-Alkoxy als gegebenenfalls vorhandene Substituenten sind beispielsweise Methyl, Ethyl, Propyl, Isopropyl, Butyl, Isobutyl, Methoxy, Ethoxy, Propoxy, Isopropoxy, Butoxy, Isobutoxy. Von diesen sind Methyl, Ethyl, Methoxy und Ethoxy als gegebenenfalls vorhandene Substituenten bevorzugt. In besonders bevorzugter Weise haben die Substituenten die Bedeutung Methyl oder Methoxy. In weiterhin bevorzugter Weise ist. R² Wasserstoff, während R¹ den genannten Bedeutungsumfang annehmen kann. In ganz besonders bevorzugter Weise wird nichtsubstituiertes Anilin zu nichtsubstituiertem Cyclohexylamin und nicht substituiertem Dicyclohexylamin hydriert.

Die nach der Hydrierung erhaltenen Reaktionsgemische enthalten kein Cyclohexan, sofern dieses nicht als Lösungsmittel hinzugefügt wurde, so daß besonders hohe Gehalte an gegebenenfalls substituiertem Cyclohexylamin und gegebenenfalls substituiertem Dicyclohexylamin erzielt werden können. Die Hydriergemische können durch einfache Destillation aufgearbeitet werden. Für eine solche Aufarbeitung kann es vorteilhaft sein, das gegebenenfalls substituierte Anilin nicht vollständig umzusetzen. Das nicht vollständig umgesetzte Anilin kann wieder in die Reaktion zurückgeführt werden. Auch der nichtverbrauchte Anteil des in 10-80fachem molaren Überschuß zugesetzten Wasserstoffs kann in die Reaktion zurückgeführt werden, wobei in vorteilhafter Weise der größte Teil dieses nicht umgesetzten Wasserstoffs in einem Hochdruckabscheider zurückgewonnen wird, so daß die Kompressionsarbeit für den Wasserstoff nicht noch einmal geleistet zu werden braucht.

Das erfindungsgemäß hergestellte gegebenenfalls substituierte Cyclohexylamin und das gegebenenfalls substituierte Dicyclohexylamin werden nach erfolgter destillativer Trennung in einer Reinheit von mindestens 99,9 Gew.-% erhalten. In dieser Reinheit sind die genannten Verbindungen in der Regel für alle weiterverarbeitenden Prozesse einsetzbar.

Die Variabilität des erfindungsgemäßen Verfahrens zeigt sich in einer starken Zunahme des Anteils an gegebenenfalls substituiertem Dicyclohexylamin gegenüber dem gegebenenfalls substituiertem Cyclohexylamin mit steigender Temperatur unter sonst gleichen Bedingungen. So erhält man beispielsweise eine Steigerung des Anteils an gegebenenfalls substituiertem Dicyclohexylamin im Temperaturbereich von etwa 200-240°C auf das 1,5-5fache des Anteils im Temperaturbereich von 140-180°C. Im Bereich von etwa 185-210°C bleibt das Verhältnis zwischen gegebenenfalls substituiertem Cyclohexylamin und gegebenenfalls substituiertem Dicyclohexylamin auch während einer längeren Reaktionsdauer innerhalb enger Grenzen nahezu gleich und beträgt etwa Cyclohexylamin:Dicyclohexylamin = 1,5-6:1.

### Beispiel 1

Ein senkrecht stehendes, wärmeisoliertes Hochdruckrohr aus nichtrostendem, säurefestem Stahl von 45 mm Innendurchmesser und 1 m Länge, das vorher mit Stickstoff sauerstofffrei gespült worden war, wurde mit 1,4 l eines durch Tablettierung von Pulvern von Kobalt-, Mangan-, Kupfer- und Barium-Oxiden hergestellten Hydrierungskatalysators gefüllt. Der Kobaltgehalt der Tabletten lag bei 53 Gew.-%, der Mangangehalt bei 14 Gew.-%, der Kupfergehalt bei 0,2 Gew.-% und der Bariumgehalt bei 0,9 Gew.-% (Rest zu 100 Gew.-%: Sauerstoff). Die Tabletten hatten bei einer Zylinderhöhe von 6 mm und einem Durchmesser von 6 mm eine Druckfestigkeit von 553 N/cm² auf die Querschnittsfläche und von 156 N, gemessen als Kraft, auf die Zylindermantelfläche sowie eine innere Oberfläche von 138 m²/g.

Diesem ersten Hochdruckrohr wurde über eine feste Hochdruckleitung ein zweites mit Stickstoff gespültes, senkrecht stehendes, wärmeisoliertes Hochdruckrohr aus nichtrostendem, säurefestem Stahl von 45 mm Innendurchmesser und 1 m Länge nachgeschaltet, das mit 1,4 l eines durch Tablettierung von Pulvern von Kobalt-, Mangan-, Silicium- und Magnesium-Oxiden hergestellten Hydrierungskatalysators gefüllt worden war. Der Kobaltgehalt der Tabletten lag bei 41 Gew.-%, der Mangangehalt bei 4,1 Gew.-%, der Siliciumgehalt bei 9,3 Gew.-% und der Magnesiumgehalt bei 4,1 Gew.-% (Rest zu 100 Gew.-%: Sauerstoff). Die Tabletten hatten bei einer Zylinderhöhe von 3 mm und einem Durchmesser von 6 mm eine Druckfestigkeit von 332 N/cm² auf die Querschnittsfläche und von 100 N, gemessen als Kraft auf die Zylindermantelfläche sowie eine innere Oberfläche von 168 m²/g.

Zur Aktivierung der beiden unterschiedliche Mischungen von Metalloxiden enthaltenden Katalysatoren wurden die Tabletten gemeinsam zunächst 6 Stunden im Stickstoffstrom getrocknet (Temperatur: max. 200°C, Menge: 5 Nm³ N₂/h). Die eigentliche Aktivierung erfolgte unter einem Stickstoffdruck von 200 bar bei einer Temperatur zwischen 180 und 200°C, wobei dem Inertgas allmählich Wasserstoff zugemischt wurde, dessen Zumischungsanteil in der Anfangsphase 10-15 Vol.-% nicht übersteigen durfte. Innerhalb von 24 Stunden wurde der Stickstoffanteil der Gasmischung mehr und mehr vermindert, bis schließlich reiner Wasserstoff den Reaktor durchströmte. Die Reaktion war beendet, wenn kein Reaktionswasser, das in einem den Reaktoren nachgeschalteten Abscheider gesammelt wurde, mehr gebildet wurde.

Nach Aktivierung der Hydrierkatalysatoren wurde der Wasserstoffdruck in den beiden Reaktorsystemen, die so miteinander verknüpft waren, daß das zu hydrierende Anilin beide Hochdruckrohre von oben nach unten absteigend passieren mußte, auf 300 bar erhöht. Anschließend wurden stündlich 560 g Anilin gemeinsam mit 10 Nm³ Wasserstoff unter einem Druck von 300 bar durch die hintereinandergeschalteten Hochdruckrohre gepumpt, wobei das Anilin vor Eintritt in das erste Hochdruckrohr in einem vorgeschalteten elektrisch beheizten Wärmeaustauscher auf eine Temperatur von 160°C erhitzt wurde. Das das zweite Reaktionsrohr verlassende Reaktionsprodukt wurde in einem zweiten Wärmeaustauscher (Wasserkühler) unter 300 bar Wasserstoffdruck auf eine Temperatur <60°C abgekühlt und in einem Gasabscheider vom überschüssigen Wasserstoff, der wieder in das Hydriersystem zurückgeführt werden konnte, getrennt. Nach weiterer Abkühlung auf eine Temperatur <30°C und Entspannung auf Normaldruck wurde das Reaktionsprodukt gaschromatographisch untersucht. Bei stationären Reaktionsbedingungen ergab sich folgende Produktzusammensetzung in Abhängigkeit von den Reaktionstemperaturen (Angabe in Flächen-(F-)%; der Rest zu 100 % sind Anilin und Nebenprodukte).

| **Laufzeit (h)** | **Temperatur °C** | **CHA*****)** **(F-%)** | **DCHA*****)** **(F-%)** |
|---|---|---|---|
| 48 | 170 | 72,8 | 22,4 |
| 98 | 180 | 69,4 | 27,2 |
| 418 | 190 | 64,5 | 33,4 |
| 588 | 200 | 55,4 | 42,8 |
| 742 | 210 | 53,4 | 45,4 |
| 812 | 220 | 46,8 | 53,1 |

| | | | |
|---|---|---|---|
| *) CHA = Cyclohexylamin, DCHA = Dicyclohexylamin | | | |

### Beispiel 2

Ein senkrecht stehendes, wärmeisoliertes Hochdruckrohr aus nichtrostendem, säurefestem Stahl von 90 mm Innendurchmesser und 1,8 m Länge, das vorher mit Stickstoff sauerstofffrei gespült worden war, wurde zunächst mit 10,26 l eines durch Tablettierung von Pulvern von Kobalt-, Mangan-, Silicium- und Magnesium-Oxiden hergestellten Hydrierungskatalysators gefüllt. Der Kobaltgehalt der Tabletten lag bei 35 Gew.-%, der Mangangehalt der Tabletten bei 4,9 Gew.-%, der Siliciumgehalt bei 11 Gew.-% und der Magnesiumgehalt bei 3,0 Gew.-% (Rest zu 100 Gew.-%: Sauerstoff). Die Tabletten hatten bei einer Zylinderhöhe von 3 mm und einem Durchmesser von 6 mm eine Druckfestigkeit von 348 N/cm² auf die Querschnittsfläche und eine Druckfestigkeit von 100 N, gemessen als Kraft auf die Zylindermantelfläche sowie eine innere Oberfläche von 148 m²/g.

Anschließend wurden auf diesen Katalysator 1,14 l eines durch Tablettierung von Pulvern von Kobalt-, Mangan-, Kupfer- und Barium-Oxiden hergestellten Hydrierungskatalysators gefüllt. Der Kobaltgehalt der Tabletten lag bei 52 Gew.-%, der Mangangehalt bei 16 Gew.-%, der Kupfergehalt bei 0,18 Gew.-% und der Bariumgehalt bei 0,91 Gew.-% (Rest zu 100 %: Sauerstoff). Die Tabletten hatten bei einer Zylinderhöhe von 7 mm und einem Durchmesser von 7 mm eine Druckfestigkeit von 420 N/cm² auf die Querschnittsfläche und von 160 N auf die Zylindermantelfläche sowie eine innere Oberfläche von 180 m²/g.

Nach Aktivierung der oxidischen Hydrierkatalysatoren wie in Beispiel 1 wurde der Wasserstoffdruck auf 300 bar erhöht. Anschließend wurden stündlich in der Rieselphase 2 280 g Anilin gemeinsam mit 70 Nm³ Wasserstoff unter einem Druck von 300 bar durch das Hochdruckrohr gepumpt, wobei das zu hydrierende Anilin vor Eintritt in das Hochdruckrohr in einem vorgeschalteten elektrisch beheizten Wärmeaustauscher auf eine Temperatur von 160°C erhitzt wurde. Das das Reaktionsrohr verlassende Reaktionsprodukt wurde in einem zweiten Wärmeaustauscher (Wasserkühler) unter 300 bar Wasserstoffdruck auf eine Temperatur <60°C abgekühlt und in einem Gasabscheider vom überschüssigen Wasserstoff, der wieder in das Hydriersystem zurückgeführt wurde, getrennt. Nach weiterer Abkühlung auf eine Temperatur <30°C und Entspannung auf Normaldruck wurde das Reaktionsprodukt gaschromatographisch untersucht. Bei stationären Reaktionsbedingungen ergab sich folgende Produktzusammensetzung in Abhängigkeit von den Reaktionstemperaturen (der Rest zu 100 % sind Anilin und Nebenprodukte):

| **Laufzeit (h)** | **Temperatur °C** | **CHA*****)** **(F-%)** | **DCHA*********) (F-%)** |
|---|---|---|---|
| 48 | 160 | 70,2 | 25,4 |
| 96 | 170 | 60,5 | 35,7 |
| 128 | 180 | 47,4 | 50,8 |
| 220 | 190 | 38,5 | 60,6 |
| 341 | 200 | 30,2 | 69,2 |
| 412 | 210 | 26,0 | 73,4 |
| 514 | 220 | 18,4 | 81,2 |

| | | | |
|---|---|---|---|
| *) CHA = Cyclohexylamin, DCHA = Dicyclohexylamin | | | |

### Beispiel 3

Ein senkrecht stehendes, wärmeisoliertes Hochdruckrohr aus nichtrostendem, säurefestem Stahl von 90 mm Innendurchmesser und 1,8 m Länge, das vorher mit Stickstoff sauerstofffrei gespült worden war, wurde zunächst mit 1,14 l eines durch Tablettierung von Pulvern von Kobalt-, Mangan-, Silicium- und Magnesium-Oxiden hergestellten Hydrierungskatalysators gefüllt. Der Kobaltgehalt der Tabletten lag bei 35 Gew.-%, der Mangangehalt der Tabletten bei 4,9 Gew.-%, der Siliciumgehalt bei 11 Gew.-% und der Magnesiumgehalt bei 3,0 Gew.-% (Rest zu 100 Gew.-%: Sauerstoff). Die Tabletten hatten bei einer Zylinderhöhe von 3 mm und einem Durchmesser von 6 mm eine Druckfestigkeit von 348 N/cm² auf die Querschnittsfläche und eine Druckfestigkeit von 100 N, gemessen als Kraft auf die Zylindermantelfläche sowie eine innere Oberfläche von 148 m²/g.

Anschließend wurden auf diesen Katalysator 11,26 l eines durch Tablettierung von Pulvern von Kobalt-, Mangan-, Kupfer- und Barium-Oxiden hergestellten Hydrierungskatalysators gefüllt. Der Kobaltgehalt der Tabletten lag bei 53 Gew.-%, der Mangangehalt bei 14 Gew.-%, der Kupfergehalt bei 0,2 Gew.-% und der Bariumgehalt bei 0,9 Gew.-% (Rest zu 100 %: Sauerstoff). Die Tabletten hatten bei einer Zylinderhöhe von 7 mm und einem Druchmesser von 7 mm eine Druckfestigkeit von 420 N/cm² auf die Querschnittsfläche und von 160 N auf die Zylindermantelfläche sowie eine innere Oberfläche von 180 m²/g.

Nach Aktivierung der oxidischen Hydrierkatalysatoren wie in Beispiel 1 wurde der Wasserstoffdruck auf 300 bar erhöht. Anschließend wurden stündlich in der Rieselphase 2 280 g Anilin gemeinsam mit 70 Nm³ Wasserstoff unter einem Druck von 300 bar durch das Hochdruckrohr gepumpt, wobei das zu hydrierende Anilin vor Eintritt in das Hochdruckrohr in einem vorgeschalteten elektrisch beheizten Wärmeaustauscher auf eine Temperatur von 160°C erhitzt wurde. Das das Reaktionsrohr verlassende Reaktionsprodukt wurde in einem zweiten Wärmeaustauscher (Wasserkühler) unter 300 bar Wasserstoffdruck auf eine Temperatur <60°C abgekühlt und in einem Gasabscheider vom überschüssigen Wasserstoff, der wieder in das Hydriersystem zurückgeführt werden konnte, getrennt. Nach weiterer Abkühlung auf eine Temperatur <30°C und Entspannung auf Normaldruck wurde das Reaktionsprodukt gaschromatographisch untersucht. Bei stationären Reaktionsbedingungen ergab sich folgende Produktzusammensetzung in Abhängigkeit von den Reaktionstemperaturen (der Rest zu 100 % sind Anilin und Nebenprodukte):

| **Laufzeit (h)** | **Temperatur** °**C** | **CHA*****)** **(F-%)** | **DCHA*****)** **(F-%)** |
|---|---|---|---|
| 48 | 180 | 82,2 | 9,89 |
| 96 | 190 | 85,4 | 12,70 |
| 114 | 200 | 90,6 | 9,3 |
| 136 | 210 | 84,9 | 14,6 |
| 224 | 220 | 81,2 | 18,2 |
| 364 | 230 | 74,2 | 24,4 |
| 568 | 240 | 70,2 | 28,2 |

| | | | |
|---|---|---|---|
| *) CHA = Cyclohexylamin, DCHA = Dicyclohexylamin | | | |

### Beispiel 4

Ein senkrecht stehendes, wärmeisoliertes Hochdruckrohr aus nichtrostendem, säurefestem Stahl von 90 mm Innendurchmesser und 1,8 m Länge, das vorher mit Stickstoff sauerstofffrei gespült worden war, wurde zunächst mit 4,6 l eines durch Tablettierung von Pulvern von Kobalt-, Mangan-, Silicium- und Magnesium-Oxiden hergestellten Hydrierungskatalysators gefüllt. Der Kobaltgehalt der Tabletten lag bei 41 Gew.-%, der Mangangehalt bei 5,4 Gew.-%, der Siliciumgehalt bei 9,3 Gew.-% und der Magnesiumgehalt bei 4,1 Gew.-% (Rest zu 100 Gew.-%: Sauerstoff). Die Tabletten hatten bei einer Zylinderhöhe von 6 mm und einem Durchmesser von 8 mm eine Druckfestigkeit von 305 N/cm² auf die Querschnittsfläche und eine Druckfestigkeit von 100 N, gemessen als Kraft auf die Zylindermantelfläche sowie eine innere Oberfläche von 165 m²/g.

Anschließend wurden auf diesen Katalysator 6,8 l eines durch Tablettierung von Pulvern von Kobalt-, Mangan-, Kupfer- und Barium-Oxiden hergestellten Hydrierungskatalysators gefüllt. Der Kobaltgehalt der Tabletten lag bei 53 Gew.-%, der Mangangehalt bei 14 Gew.-%, der Kupfergehalt bei 0,2 Gew.-% und der Bariumgehalt bei 0,9 Gew.-%. Die Tabletten hatten bei einer Zylinderhöhe von 7 mm und einem Durchmesser von 7 mm eine Druckfestigkeit von 420 N/cm² auf die Querschnittsfläche und von 160 N auf die Zylindermantelfläche sowie eine innere Oberfläche von 180 m²/g.

Nach Aktivierung der oxidischen Hydrierkatalysatoren wie in Beispiel 1 wurde der Wasserstoffdruck auf 300 bar erhöht. Anschließend wurden stündlich in der Rieselphase 2 280 g Anilin gemeinsam mit 70 Nm³ Wasserstoff unter einem Druck von 300 bar durch das Hochdruckrohr gepumpt, wobei das zu hydrierende Anilin vor Eintritt in das Hochdruckrohr in einem vorgeschalteten elektrisch beheizten Wärmeaustauscher auf eine Temperatur von 200°C erhitzt wurde. Das das Reaktionsrohr verlassende Reaktionsprodukt wurde in einem zweiten Wärmeaustauscher (Wasserkühler) unter 300 bar Wasserstoffdruck auf eine Temperatur <60°C abgekühlt und in einem Gasabscheider vom überschüssigen Wasserstoff, der wieder in das Hydriersystem zurückgeführt wurde, getrennt. Nach weiterer Abkühlung auf eine Temperatur <30°C und Entspannung auf Normaldruck wurde das Reaktionsprodukt gaschromatographisch untersucht. Bei stationären Reaktionsbedingungen ergab sich folgende Produktzusammensetzung (der Rest zu 100 % sind Anilin und Nebenprodukte):

| **Laufzeit (h)** | **Temperatur °C** | **CHA*****)** **(F-%)** | **DCHA*****)** **(F-%)** |
|---|---|---|---|
| 163 | 199 | 73,64 | 25,57 |
| 3.828 | 197 | 73,01 | 26,06 |
| 6.114 | 185 | 73,80 | 22,86 |
| 9.062 | 210 | 76,93 | 21,00 |
| 16.462 | 185 | 71,16 | 22,62 |
| 19,805 | 195 | 73,50 | 22,70 |
| 25.167 | 210 | 72,51 | 23,10 |

| | | | |
|---|---|---|---|
| *) CHA = Cyclohexylamin, DCHA = Dicyclohexylamin | | | |

## Patentansprüche

1. Verfahren zur Herstellung eines Gemisches aus Cyclohexylamin und Dicyclohexylamin der Formeln 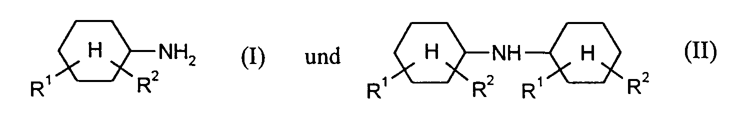 durch katalytische Hydrierung von Anilin der Formel 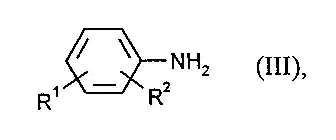 wobei in den Formeln
R¹ und R² unabhängig voneinander Wasserstoff, C₁-C₄-Alkyl oder C₁-C₄-Alkoxy bedeuten,
bei einer Reaktionstemperatur von 140 bis 260°C und einem H₂-Druck von 10 bis 400 bar, dadurch gekennzeichnet, daß ein System aus zwei reduzierten, trägerlosen Katalysatoren A + B aus verpreßten Element(hydr)oxid-Pulvern eingesetzt wird, wobei folgende Elementanteile enthalten sind:
für Katalysator A: 40-60 Gew.-% eines oder mehrere von Fe, Co, Ni;
10-20 Gew.-% Mn; 0,05-1,5 Gew.-% Cu; und
0,2-5 Gew.-% eines oder mehrere von Ca, Sr, Ba; und
für Katalysator B: 30-50 Gew.-% eines oder mehrere von Fe, Co, Ni; 3-10 Gew.-% Mn; 5-15 Gew.-% Si; und 2-8 Gew.-% Mg;
und wobei in A und in B der Rest zu 100 Gew.-% Sauerstoff darstellt und die Prozentangaben auf das Gesamtgewicht des jeweiligen Katalysators A oder B bezogen sind.

2. Verfahren nach Anspruch 1, dadurch gekennzeichnet, daß aus der Gruppe Fe, Co, Ni das Co oder das Ni oder ein Gemisch Co/Ni, bevorzugt das Co eingesetzt wird.

3. Verfahren nach Anspruch 1, dadurch gekennzeichnet, daß aus der Gruppe Ca, Sr, Ba das Sr oder das Ba oder ein Gemisch Sr/Ba, bevorzugt das Ba eingesetzt wird.

4. Verfahren nach Anspruch 1, dadurch gekennzeichnet, daß die Katalysatoren A + B eine Druckfestigkeit von 200-800 N/cm², bevorzugt 250-600 N/cm², auf ebene Preßoberflächen, eine Druckfestigkeit von 50-200 N, gemessen als Kraft, bevorzugt 80-140 N, auf gewölbte Preßoberflächen und eine innere Oberfläche von 30-200 m²/g, bevorzugt 80-160 m²/g, aufweisen.

5. Verfahren nach Anspruch 1, dadurch gekennzeichnet, daß die Katalysatoren A + B im Gewichtsverhältnis von 0,5 A:9,5 B bis 9,5 A:0,5 B eingesetzt werden.

6. Verfahren nach Anspruch 1, dadurch gekennzeichnet, daß kontinuierlich in der Rieselphase an fest angeordneten Katalysatoren gearbeitet wird und eine Katalysatorbelastung von 0,1-3 kg, bevorzugt von 0,15-1,5 kg, Anilin pro Liter Katalysator und Stunde eingestellt wird.

7. Verfahren nach Anspruch 1, dadurch gekennzeichnet, daß bei einem H₂-Druck von 20-350 bar, bevorzugt 100-300 bar, gearbeitet wird.

8. Verfahren nach Anspruch 1, dadurch gekennzeichnet, daß bei einer Temperatur von 160-230°C gearbeitet wird.

9. Verfahren nach Anspruch 1, dadurch gekennzeichnet, daß die Katalysatoren A und B vor ihrem Einsatz getrennt oder gemeinsam durch Behandlung mit Wasserstoff bei 160-240°C und 10-400 bar reduziert werden, wobei der Wasserstoff zu Beginn der Reduktion als H₂/Inertgas-Gemisch eingesetzt wird und der Inertgasanteil im Verlauf der Reduktion vollständig entfernt wird.

10. Verfahren nach Anspruch 1, dadurch gekennzeichnet, daß das gegebenenfalls substituierte Anilin mit 10-100 Gew.-%, bevorzugt 10-40 Gew.-%, eines reaktionsinerten Lösungsmittels, bezogen auf das gegebenenfalls substituierte Anilin, verdünnt wird.

## Claims

1. Process for preparing a mixture of cyclohexylamine and dicyclohexylamine of the formulae 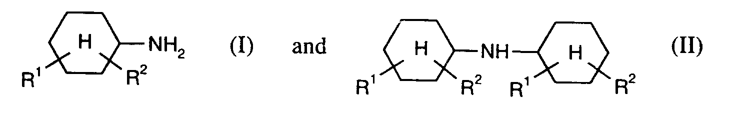 by catalytic hydrogenation of aniline of the formula 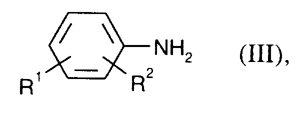 where, in the formulae,
R¹ and R² are, independently of one another, hydrogen, C₁-C₄-alkyl or C₁-C₄-alkoxy,
at a reaction temperature of from 140 to 260°C and an H₂ pressure of from 10 to 400 bar, characterized in that the catalyst system used comprises two reduced, unsupported catalysts A + B comprising pressed element (hydr)oxide powders in which the following element proportions are present:
for catalyst A: 40-60% by weight of one or more of Fe, Co, Ni;
10-20% by weight of Mn;
0.05-1.5% by weight of Cu;
and
0.2-5% by weight of one or more of Ca, Sr, Ba; and
for catalyst B: 30-50% by weight of one or more of Fe, Co, Ni; 3-10% by weight of Mn; 5-15% by weight of Si; and 2-8% by weight of Mg;
where, in A and B, the remainder to 100% by weight is oxygen and the percentages are based on the total weight of the respective catalyst A or B.

2. Process according to Claim 1, characterized in that the element from the group consisting of Fe, Co and Ni which is used is Co or Ni or a Co/Ni mixture, preferably Co.

3. Process according to Claim 1, characterized in that the element from the group consisting of Ca, Sr and Ba which is used is Sr or Ba or an Sr/Ba mixture, preferably Ba.

4. Process according to Claim 1, characterized in that the catalysts A and B have a compressive strength of 200-800 N/cm², preferably 250-600 N/cm², on flat pressed surfaces, a compressive strength of 50-200 N, measured as force, preferably 80-140 N, on curved pressed surfaces and an internal surface area of 30-200 m²/g, preferably 80-160 m²/g.

5. Process according to Claim 1, characterized in that the catalysts A and B are used in a weight ratio of from 0.5 A:9.5 B to 9.5 A:0.5 B.

6. Process according to Claim 1, characterized in that the reaction is carried out continuously in the downflow mode over fixed-bed catalysts and the weight hourly velocity is 0.1-3 kg, preferably 0.15-1.5 kg, of aniline per litre of catalyst and hour.

7. Process according to Claim 1, characterized in that the reaction is carried out at an H₂ pressure of 20-350 bar, preferably 100-300 bar.

8. Process according to Claim 1, characterized in that the reaction is carried out at a temperature of 160-230°C.

9. Process according to Claim 1, characterized in that the catalysts A and B are, prior to use, reduced separately or together by treatment with hydrogen at 160-240°C and 10-400 bar, with the hydrogen being used as H₂/inert gas mixture at the beginning of the reduction and the proportion of inert gas being completely removed during the course of the reduction.

10. Process according to Claim 1, characterized in that the unsubstituted or substituted aniline is diluted with 10-100% by weight, preferably 10-40% by weight, of a reaction-inert solvent, based on the unsubstituted or substituted aniline.

## Revendications

1. Procédé pour la préparation d'un mélange de cyclohexylamine et de dicyclohexylamine de formules 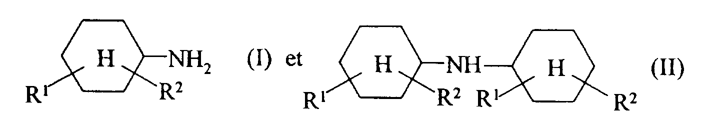 par hydrogénation catalytique d'une aniline de formule 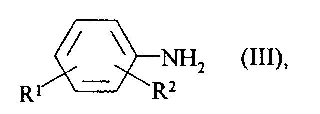 dans lesquelles
R¹ et R² représentent chacun, indépendamment l'un de l'autre, l'hydrogène, un groupe alkyle en C₁-C₄ ou alcoxy en C₁-C₄,
à une température de réaction de 140 à 260°C et une pression d'hydrogène de 10 à 400 bar, caractérisé en ce que l'on utilise un système de deux catalyseurs réduits, sans support, A + B, consistant en poudres comprimées d'(hydr)oxydes d'éléments, et contenant les éléments en question aux proportions suivantes :
pour le catalyseur A :
40 à 60 % en poids d'un ou plusieurs des éléments Fe, Co, Ni ; 10 à 20 % en poids de Mn ; 0,05 à 1,5% en poids de Cu ; et 0,2 à 5 % en poids d'un ou plusieurs des éléments Ca, Sr, Ba ; et
pour le catalyseur B :
30 à 50 % en poids d'un ou plusieurs des éléments Fe, Co, Ni ; 3 à 10 % en poids de Mn ; 5 à 15 % en poids de Si ; et 2 à 8 % en poids de Mg ;
le complément à 100 % en poids de A et B consistant en oxygène, et les pourcentages indiqués se rapportant au poids total du catalyseur A et respectivement du catalyseur B.

2. Procédé selon la revendication 1, caractérisé en ce que, dans le groupe Fe, Co, Ni, on utilise Co ou Ni ou un mélange Co/Ni, de préférence Co.

3. Procédé selon la revendication 1, caractérisé en ce que, dans le groupe Ca, Sr, Ba, on utilise Sr ou Ba ou un mélange Sr/Ba, de préférence Ba.

4. Procédé selon la revendication 1, caractérisé en ce que les catalyseurs A + B ont une résistance à la compression de 200 à 800 N/cm², de préférence de 250 à 600 N/cm², sur les surfaces comprimées planes, une résistance à la compression de 50 à 200 N, mesure en force, de préférence de 80 à 140 N, sur les surfaces comprimées courbes, et une surface interne de 30 à 200 m²/g, de préférence de 80 à 160 m²/g.

5. Procédé selon la revendication 1, caractérisé en ce que les catalyseurs A + B sont mis en oeuvre à des proportions relatives en poids de 0,5 A:9,5 B à 9,5 A:0,5 B.

6. Procédé selon la revendication 1, caractérisé en ce que l'on opère en continu, en phase ruisselante, sur des catalyseurs en disposition fixe et on règle à une charge des catalyseurs de 0,1 à 3 kg, de préférence de 0,15 à 1,5 kg d'aniline par litre de catalyseur et par heure.

7. Procédé selon la revendication 1, caractérisé en ce que l'on opère à une pression d'hydrogène de 20 à 350 bar, de préférence de 100 à 300 bar.

8. Procédé selon la revendication 1, caractérisé en ce que l'on opère à des températures de 160 à 230°C.

9. Procédé selon la revendication 1, caractérisé en ce que, avant leur utilisation, les catalyseurs A et B sont réduits, séparément ou ensemble, par traitement à l'hydrogène à des températures de 160 à 240°C sous des pressions de 10 à 400 bar, l'hydrogène est mis en oeuvre au début de la réduction sous forme d'un mélange hydrogène/gaz inerte et dans le cours de la réduction, on élimine totalement la proportion de gaz inerte.

10. Procédé selon la revendication 1, caractérisé en ce que l'aniline éventuellement substituée est diluée par 10 à 100 % de son poids, de préférence 10 à 40 % de son poids, d'un solvant inerte dans la réaction.
